# EUROPÄISCHE PATENTSCHRIFT

(11) **EP 3 159 041 B1**
(45) Veröffentlichungstag und Bekanntmachung des Hinweises auf die Patenterteilung: **12.08.2020**
(21) Anmeldenummer: 16194870.8
(22) Anmeldetag: 20.10.2016
(51) Int. Cl.: A61N 5/06

(54) **SYSTEM, VERFAHREN SOWIE COMPUTERPROGRAMM ZUR STIMULIERUNG VON NEURONALEN STRUKTUREN**
SYSTEM, METHOD, AND COMPUTER PROGRAM FOR STIMULATION OF NEURAL STRUCTURES
SYSTÈME, PROCÉDÉ ET PROGRAMME D'ORDINATEUR DESTINÉS À STIMULER DES STRUCTURES NEURONALES

(30) Priorität: 20.10.2015 DE 102015117830
(43) Veröffentlichungstag der Anmeldung: 26.04.2017
(73) Patentinhaber: Baumann, Gottfried Bruno, 47546 Kalkar (DE)
(72) Erfinder: Baumann, Gottfried Bruno, 47546 Kalkar (DE)
(74) Vertreter: Vogel, Andreas

(56) Entgegenhaltungen:
- EP-A1- 1 642 609
- EP-A2- 2 098 261
- DE-U1-202012 002 256

## Beschreibung

Die vorliegende Erfindung betrifft ein System zur Stimulierung von neuronalen Strukturen gemäß dem Oberbegriff von Anspruch 1 sowie ein Verfahren zur Stimulierung von neuronalen Strukturen nach dem Oberbegriff des unabhängigen Anspruchs 11 und ein Computerprogramm für ein System zur Stimulierung von neuronalen Strukturen gemäß dem Oberbegriff des unabhängigen Anspruchs 14.

Systeme zur Stimulierung von neuronalen Strukturen sind grundsätzlich bekannt. Nachteiligerweise hat sich herausgestellt, dass beim Stimulieren neuronaler Strukturen häufig nicht die gewünschten Ergebnisse erzielt werden, da mit mehrfachen Stimulierungen immer mehr Energie aufgewendet werden muss, um den gewünschten Effekt zu erzielen. Ferner hat sich der Nachteil herausgestellt, dass bestimmte neuronale Strukturen nicht gezielt stimuliert werden können.

In der Schrift EP 2 098 261 A1 ist ebenfalls eine Vorrichtung und ein Verfahren zur visuellen Stimulation mit maximalen Pulsdauern von 1 sec mittels einer Steuereinheit und einer Stimulationseinheit offenbart.

Es ist daher die Aufgabe der vorliegenden Erfindung, ein System sowie ein Verfahren und ein Computerprogramm bereitzustellen, welche mindestens einen Nachteil aus dem Stand der Technik zumindest zum Teil überwindet. Insbesondere ist die Aufgabe der Erfindung ein System bereitzustellen, welches Habituationseffekte neuronaler Strukturen verhindert und insbesondere den Wirkungsgrad und die Präzision der Stimulierung neuronaler Strukturen erhöht. Zur Lösung dieser Aufgabe wird ein System zur Stimulierung von neuronalen Strukturen mit den technischen Merkmalen des Anspruchs 1 sowie ein Verfahren nicht therapeutischen Stimulierung von neuronalen Strukturen mit den technischen Merkmalen des Anspruchs 11 und ein Computerprogramm für ein System zur Stimulierung von neuronalen Strukturen mit den technischen Merkmalen des Anspruchs 14 vorgeschlagen, denen nachfolgende besondere Bedeutung zukommen.

Weitere Merkmale und Details der Erfindung ergeben sich aus den Unteransprüchen, der Beschreibung und den Zeichnungen. Dabei gelten Merkmale und Details, die im Zusammenhang mit dem erfindungsgemäßen System beschrieben worden sind, selbstverständlich auch im Zusammenhang mit dem erfindungsgemäßen Verfahren sowie dem erfindungsgemäßen Computerprogramm und umgekehrt, so dass bzgl. der Offenbarung zu den einzelnen Erfindungsaspekten stets wechselseitig Bezug genommen wird bzw. werden kann. Unter neuronalen Strukturen können im Rahmen der vorliegenden Erfindung neben Nervenzellen ebenfalls Muskelzellen, zur physikalischen Beeinflussung der Aktivität dieser Zellen verstanden werden. Zweckmäßige Ausgestaltungen der Erfindung sind in den abhängigen Ansprüchen aufgeführt. Dabei ist der Kern der Erfindung ein System zur Stimulierung, insbesondere zur Photostimulation, von neuronalen Strukturen zur physikalischen Beeinflussung der Aktivität der neuronalen Strukturen. Ferner umfasst das System mindestens ein Steuergerät zur Steuerung und/oder Regelung von zumindest einer Lichtquelle zur Applikation von Licht. Bei der Stimulation kann es sich insbesondere um eine Photostimulation handeln. Eine

Photostimulation kann durch Licht, insbesondere durch Lichtpulse und/oder Lichtblitze induziert werden. Die Photostimulation ist dabei als Provokationsmethode einsetzbar, bei welcher Lichtpulse und/oder Lichtblitze auf neuronale Strukturen applizierbar sind. Ein Lichtpuls weist dabei eine Dauer von 0,2 bis 200 sec, bevorzugt von 0,5 bis 100 sec und besonders bevorzugt von 1 bis 70 sec auf. Ein Lichtblitz weist dabei eine Dauer von 0,01 bis 1 sec, bevorzugt von 0,02 bis 0,5 sec und besonders bevorzugt von 0,05 bis 0,2 sec auf. Bei den neuronalen Strukturen kann es sich um einen Verbund einzelner neuronaler Zellen handeln. Neuronale Zellen können Nervenzellen sein, welche in der Lage sind, Erregungen zu übertragen. Nervenzellen weisen oftmals einen Zellkörper sowie Zellfortsätze auf, wobei die Zellfortsätze, insbesondere Dendriten, zum einen Erregungen aufnehmen und insbesondere Axone zum anderen Erregungen weiterleiten können. Die Fortleitung einer Erregung erfolgt dabei als durch lonenströme induzierte Spannungsänderungen. Neuronale Zellen können ferner Muskelzellen sein. Muskelzellen können oftmals in spindelförmiger zellulärer Struktur zusammengefasst sein und Muskelfasern bilden. Derartige Muskelfasen können bis zu mehreren hundert Myofibrillen aufweisen oftmals mit einem Durchmesser von ungefähr 1 µm. Myofibrillen können eine Muskelfaser über die gesamte Länge in paralleler Ausrichtung zueinander durchziehen. Muskelfasern weisen im Querschnitt ungefähr 0,01 mm bis 0,1 mm auf und können, je nach Art und Länge des einzelnen Muskels, ungefähr 2 mm bis ungefähr 25 cm lang sein. In der Skelettmuskulatur können sich mehrere Muskelfasern zu Faserbündeln zusammenschließen, wobei diese an Knochen und Sehnen angeordnet sein können. Diese weisen einen Durchmesser von ungefähr 0,1 mm bis 1 mm auf. Bei einer Kontraktion der Muskelfaser verkürzen sich diese signifikant. Bei einer physikalischen Beeinflussung der Aktivität von neuronalen Strukturen handelt es sich insbesondere um eine Aktivität der Strukturen aufgrund von physikalischen Stimuli, wie bspw. Licht. Eine Photostimulation kann die Aktivität neuronaler Strukturen bzw. der einzelnen Zellen steigern. Durch eine Applikation von Licht ist es möglich, dass neuronale Strukturen aktiviert werden, oder ihre Aktivität verändern. Dabei kann ebenfalls ein Verfahren genutzt werden, wobei insbesondere neuronale Strukturen, bevorzugt neuronale Strukturen wie bspw. das Gehirn, im Rhythmus ihrer elektrophysiologisch bzw. elektroenzephalographisch detektierbaren (Hirn-) Wellen durch externe Reize, bevorzugt durch Lichtpulse und/oder Lichtblitze, stimuliert werden und sich diesen externen Reizen anpassen.

Dabei kann ebenfalls ein Verfahren genutzt werden, wobei insbesondere neuronale Strukturen und damit bspw. Zellen der Skelettmuskulatur, im Rhythmus ihrer elektromyographischen detektierbaren Signale durch externe Reize, bevorzugt durch Lichtpulse und/oder Lichtblitze, stimuliert werden und sich diesen externen Reizen anpassen. Dabei ist es möglich den Effekt auf die Skelettmuskulatur elektromyographisch, insbesondere mit Oberflächenelektroden und/oder Nadelelektroden, zu erfassen, der indirekt durch die zerebralen Änderungen erwirkt werden kann, die durch die Photostimulation hervorgerufen werden können, oder der durch direkte Stimulation der Skelettmuskulatur erwirkt werden kann. Eine direkte lichterwirkte Stimulation der Muskulatur wäre bspw. nach Einbringung lichtempfindlicher Strukturen wie etwa Kanalproteine möglich. Diese lichtempfindlichen Strukturen können bspw. mittels über Glasfaser geleiteten Lichts erreicht werden und eine Muskelstimulation ermöglichen. Dabei können Effekte auf die Skelettmuskulatur im Sinne von Anspannung und Entspannung der ruhenden Muskulatur ohne willkürliche Muskelanspannung gemessen werden. Insbesondere die Amplituden und Frequenzen der elektromyographisch (EMG) erfassbaren Wellen können mit Hilfe einer Fourier Transformationsanalyse analysiert und sogenannte Power-Werte ermittelt werden. Die EMG Messungen können vorteilhafterweise vor, während und nach einer Behandlungssitzung mit Photostimulation durchgeführt werden. Insbesondere können Vergleiche zwischen den Messwerten vor und nach einer Behandlungssitzung angestellt werden. Die EMG Messungen können ferner auch im Zusammenhang mit anderen Formen der Neurostimulation als der Photostimulation durchgeführt werden. Die Lichtpulse und/oder Lichtblitze können dabei bevorzugt eine dem Aktivitätsrhythmus der neuronalen Strukturen entsprechenden Frequenz aufweisen, die beginnend mit einer niedrigen Frequenz von insbesondere 0,1 bis 50 Hz mit allmählich steigernder Frequenz beschleunigt und/oder beginnend mit einer hohen Frequenz von insbesondere 200 bis 5 Hz mit allmählich abnehmender Frequenz verlangsamt wird. Dabei ist es denkbar, dass die Lichtstimulation hinsichtlich ihres Effektes auf die Muskulatur elektromyographisch erfasst werden kann.

Bei dem Steuergerät kann es sich vorzugsweise um ein elektronisches Modul handeln. Steuergeräte arbeiten oftmals nach dem EVA-Prinzip (Eingabe-Verarbeitung-Ausgabe). Für die Eingabe werden bevorzugt Sensoren eingesetzt, welche physikalische Kenngrößen, wie z. B. Temperatur, Aktivität, usw. als Istwerte messen. Die ermittelten Werte können nachfolgend mit einer im Steuergerät gespeicherten und/oder berechneten Sollgröße verglichen werden. Falls die ermittelten Werte nicht mit den Sollwerten übereinstimmen, kann das Steuergerät die Faktoren verändern, so dass die gemessenen Istwerte wieder mit den Sollgrößen übereinstimmen. Regelung bedeutet dabei Messen der zu beeinflussenden Größe (Regelgröße) in der Regelstrecke und kontinuierliches Vergleichen mit dem gewünschten Wert (Sollwert oder Führungsgröße). Der Regler kann entsprechend der Abweichung (Regeldifferenz) eine Stellgröße bestimmen, die so auf die Regelgröße einwirkt, dass sie die Abweichung minimiert und die Regelgröße ein gewünschtes Zeitverhalten annimmt, trotz vorhandener Störgrößen.

Ferner umfasst das System mindestens eine Lichtquelle. Eine Lichtquelle kann dabei der Ort sein, von dem Licht ausgeht, insbesondere in spezifischen Wellenlängen, Frequenzen und Intensitäten. Erfindungsgemäß ist das Licht dabei seitendifferent applizierbar. Bei einer seitendifferenten Applikation wird das Licht nicht gleichmäßig über eine gesamte Fläche appliziert, sondern vielmehr unterschiedlich an zumindest zwei Seiten. Die Seitendifferenz kann sich dabei in der Frequenz, der Intensität oder auch der Wellenlänge wiederspiegeln. Eine seitendifferente Applikation kann dabei von nur einer oder auch von mehreren Lichtquellen herbeigeführt werden. Bei nur einer Lichtquelle ist es denkbar, dass verschiedene Hilfsmittel wie bspw. Spiegel oder Lichtleiter einsetzbar sind, damit die eine Lichtquelle an verschiedenen Seiten Licht unterschiedlich appliziert. Eine seitendifferente Applikation ist insbesondere bei paarigen neuronalen Strukturen denkbar, wie bspw. die Augen, die Nase, das Gehirn, das Rückenmark, die Rücken- und Halsmuskulatur und die Extremitäten. Auch bei einheitlichen Strukturen ist allerdings eine seitendifferente Applikation denkbar, um diese an verschiedenen Stellen unterschiedlich zu behandeln. Obwohl eine Applikation von Licht lediglich auf oberflächennahe neuronale Strukturen, wie Haut, Nase oder Augen wirkt, können über nervöse Verschaltungen auch zentrale neuronale Strukturen wie das Gehirn und/oder das Rückenmark mittels einer Applikation von Licht erreicht werden. Insbesondere über das Auge ist es möglich, bestimmte Regionen des Gehirns zu stimulieren. Ebenfalls ist bei einer Applikation von Licht auf neuronale Strukturen denkbar, dass insbesondere oberflächennahe neuronale Strukturen, wie Teile der Skelettmuskulatur stimuliert werden. Durch eine seitendifferente Applikation ergeben sich ferner vielfältige verschiedene Möglichkeiten an Stimulationsmustern. Dabei kann insbesondere ein Verfahren angewendet werden, bei welchem neuronale Strukturen, bevorzugt im Rhythmus ihrer elektrophysiologisch bzw. elektroenzephalographisch und/oder elektromyographisch detektierbaren Wellen bzw. Signale durch externe Reize stimuliert werden. Die Stimulation erfolgt dabei bevorzugt durch Lichtpulse und/oder Lichtblitze mit aufsteigenden und/oder absteigenden Aktivitätsrhythmen.

Erfindungsgemäß kann es vorgesehen sein, dass die zumindest eine Lichtquelle mindestens eine LED umfasst. Bei einer LED handelt es sich um eine Leuchtdiode (light-emitting diode). LEDs sind vorteilhafterweise lichtemittierende Halbleiter-Bauelemente, die bei einem Durchfluss von elektrischem Strom Licht, Infrarotstrahlung oder auch Ultraviolettstrahlung ausstrahlen können. Vorteilhafterweise sind LEDs insbesondere kostengünstig und langlebig und können zudem in verschiedenen Lichtintensitäten und Farben eingesetzt werden. Die Farbe einer LED kann dabei wesentlich vom Bandabstand des eingesetzten Halbleitermaterials abhängen. Die Farbe entspricht direkt einer bestimmten Wellenlänge λ, d. h. dem Kehrwert der Frequenz der emittierten elektromagnetischen Strahlung, multipliziert mit der Ausbreitungsgeschwindigkeit.

Ferner ist es denkbar, dass mittels der Lichtquelle Licht applizierbar ist, wobei die Applikation kontinuierlich und/oder diskontinuierlich, insbesondere sequenziell, erfolgen kann. Durch verschiedenste Variationen der Applikation von Licht auf neuronale Strukturen, ist eine große Variation an Applikationsmöglichkeiten geschaffen. Das Licht wird dabei insbesondere in kurzen Lichtpulsen und/oder noch kürzeren Lichtblitzen appliziert. Ein Lichtpuls weist dabei eine Dauer von 0,2 bis 200 sec, bevorzugt von 0,5 bis 100 sec und besonders bevorzugt von 1 bis 70 sec auf. Ein Lichtblitz weist dabei eine Dauer von 0,01 bis 1 sec, bevorzugt von 0,02 bis 0,5 sec und besonders bevorzugt von 0,05 bis 0,2 sec auf. Ebenfalls ist es denkbar, dass die Lichtquelle dimmbar sein kann. Dabei kann Licht in verschiedenen Intensitätsstärken appliziert werden. Vorteilhafterweise entsteht durch eine diskontinuierliche Applikation keine Habituation der neuronalen Strukturen, da verschiedene Stimulationsmuster erreicht werden können, die verschiedene neuronale Strukturen aktivieren. Je nach zu behandelnder Indikation bzw. je nach zu behandelnden neuronalen Strukturen kann ein anderes Muster der Applikation, bestehend aus Intensität, Frequenz oder Wellenlänge appliziert werden.

Es ist ferner denkbar, dass das Licht eine Wellenlänge, insbesondere im Bereich von 300 nm bis 1000 nm, bevorzugt im Bereich von 400 nm bis 700 nm, besonders bevorzugt im Bereich von 450 nm bis 600 nm, umfasst. Bei derartigem Licht kann es sich bevorzugt um sichtbares Licht und besonders bevorzugt um weißliches Licht handeln. Bei einer Wellenlänge von ca. 560 nm kann bspw. die Hirnstruktur des Nucleus suprachiasmaticus aktiviert werden. Bei dem Nucleus suprachiasmaticus handelt es sich um ein Kerngebiet im Gehirn, insbesondere im ventralen Hypothalamus von Säugetieren. Der Nucleus ist dabei unter dem III. Hirnventrikel und über der Kreuzung der Nervi optici (Chiasma opticum) lokalisiert. Insbesondere das Gebiet des Nucleus suprachiasmaticus kann an den die circadianen Rhythmen kontrollierenden Strukturen von Säugetiere beteiligt sein. Durch wiederholte Stimulationen, bzw. durch wiederholte Stimulationsmuster, also verschiedene Abfolgen an Applikationen von Licht bzw. Lichtpulsen und/oder Lichtblitzen, insbesondere mit verschiedenen Intensitäten, Frequenzen und Wellenlängen, können Aktivitäten und damit verbunden ebenfalls sich ausbildende Vernetzungen der neuronalen Strukturen verursacht bzw. induziert werden. Ebenfalls ist es denkbar, dass sich durch eine derartige Stimulation bestimmte Vernetzungen neuronaler Strukturen nicht ausbilden. Dementsprechend können über verschiedene Applikationen von Licht unterschiedliche Indikationen gefördert oder gehemmt werden. Insbesondere Licht der Wellenlänge von ca. 560 nm oder ca. 470 nm ist zur Stimulation von neuronalen Strukturen bei Krankheiten wie insbesondere saisonale Depression, nicht-saisonale Depression, Burnout, Schizophrenie, Demenz und/oder Krampfanfällen oder dergleichen einsetzbar.

Es kann ebenfalls erfindungsgemäß vorgesehen sein, dass die Lichtquelle eine Beleuchtungsstärke von 100 bis 15000 Lux aufweist, bevorzugt eine Beleuchtungsstärke im Bereich von 500 Lux bis 12000 Lux, besonders bevorzugt im Bereich von 9000 Lux bis 10000 Lux, umfasst ist. Auch ein Wechsel zwischen verschiedenen Intensitäten des Lichts ist möglich. Dadurch können verschiedene Indikationen der neuronalen Strukturen behandelt werden. Vorteilhafterweise tritt durch den Gebrauch verschiedener Intensitäten keine Habituation der neuronalen Strukturen auf. Verschiedene Stimulationsmuster erreichen so verschiedene neuronale Strukturen und sind je nach zu behandelndem Krankheitsbild applizierbar bzw. anwendbar. Beispiele für zu behandelnde Krankheitsbilder sind bevorzugt Depressionen und Demenzen sowie Hirnfunktionsstörungen bei Hirndurchblutungsstörungen und entzündlichen Hirnerkrankungen oder dergleichen. Ebenfalls denkbar sind Anwendungen mit programmierter Lichtstimulation bspw. bei spinaler Muskelatrophie oder Lähmungen nach einem Schlaganfall.

Gemäß der Erfindung kann es ferner vorteilhaft sein, dass die neuronalen Strukturen neuronales Gewebe aufweisen, insbesondere humane Nervenzellen. Ferner sind ebenfalls tierische Nervenzellen denkbar. Ferner kann es gemäß der Erfindung vorteilhaft sein, dass die neuronalen Strukturen Muskelzellen aufweisen, insbesondere humane Muskelzellen, bevorzugt Zellen der Skelettmuskulatur. Auch Applikationen an einem menschlichen und/oder tierischen lebenden Organismus ist möglich. Vorteilhafterweise ist das System damit nicht nur an in vitro Systemen wie bspw. Zellkulturen oder extrahiertem Gewebe einsetzbar, sondern auch an peripheren neuronalen Strukturen lebender Organismen. Zudem können auch zentral nervöse Strukturen mit dem erfindungsgemäßen System aktiviert werden. Weiterhin ist es möglich, dass Muskelzellen, insbesondere der Skelettmuskulatur mit dem erfindungsgemäßen System aktiviert werden. Ferner ist es ebenfalls denkbar, dass die Stimulierung von neuronalen Strukturen bei mehreren neuronalen Strukturen oder auch bei neuronalen Strukturen mehrerer Organismen gleichzeitig stattfinden kann. Dabei kann entweder ein System mehrere Applikationsbereiche aufweisen oder stattdessen bzw. zusätzlich können ebenfalls mehrere Systeme parallel und/oder in Reihe geschaltet werden. Eine sehr effiziente Anwendung des Systems auf mehrere neuronale Strukturen gleichzeitig ist damit gewährleistet.

Des Weiteren kann die Erfindung vorsehen, dass zumindest eine Lichtquelle an einem tragbaren Element angeordnet ist, wobei das Element mindestens einen Applikationsbereich, insbesondere mit zwei Okularen, und mindestens ein Halteelement aufweist. Bei dem tragbaren Element kann es sich insbesondere um eine Brille und/oder eine Manchette handeln. Die Brille kann dabei wie eine herkömmliche Brille ausgestaltet sein und zwei Applikationsbereiche oder auch lediglich nur einen insbesondere durchgehenden Applikationsbereich aufweisen. Insbesondere kann es sich bei dem geteilten Applikationsbereich um Okulare handeln. Bevorzugt sind optische Strukturen, insbesondere Augen, durch diese Fläche abdeckbar. Es ist ebenfalls denkbar, dass es sich nicht um eine Brille, sondern eine Manschette handelt, welche an verschiedenen neuronalen Strukturen angeordnet werden kann, bzw. diese bedeckt. Da es sich bei den neuronalen Strukturen vorzugsweise um paarige Strukturen handelt, ist die Fläche insbesondere paarig ausgestaltet. Wenn es sich bei dem tragbaren Element um eine Brille handelt, weist diese bevorzugt zwei Okulare auf, wobei jedes Okular vor einem Auge angeordnet sein kann. Ferner kann das tragbare Element ein Halteelement aufweisen, welches das tragbare Element am jeweiligen Applikationsort halten kann. Bei dem Halteelement kann es sich um einen oder mehrere Bügel, insbesondere bei einer Brille, handeln, oder auch um eine Struktur, wie ein Gummiband, einen Riemen oder ähnliches, insbesondere bei einer Manschette. Das Halteelement sorgt dafür, dass das tragbare Element sicher am Applikationsort gehalten werden kann. Ferner kann das Steuergerät einzeln und mit dem tragbaren Element verbunden und/oder mit diesem zumindest teilweise zusammen verbaut sein. Dabei kann es sich um ein einteiliges Element oder auch um mehrere zusammensteckbare Teile handeln. Es ist ferner denkbar, dass das tragbare Element an sich das Steuerelement ist. Unabhängig vom tragbaren Element bzw. vom Applikationsbereich, also unabhängig davon, ob dieser einteilig oder mehrteilig aufgebaut ist, ist eine seitendifferente Applikation möglich. Ferner ist eine sequenzielle Applikation möglich. Der Applikationsbereich kann dabei ferner einen bestimmten Abstand zum Applikationsort aufweisen. Dieser Abstand kann insbesondere durch das Halteelement und/oder andere strukturelle Elemente konstant gehalten werden. Zudem ist es denkbar, dass der Applikationsbereich vor weiteren Umwelteinflüssen, wie insbesondere weiteren Lichtquellen abgedichtet ist, so dass lediglich das applizierte Licht an den Applikationsort gelangt. Vorteilhafterweise kann das tragbare Element in einfacher Weise zu den neuronalen Strukturen angeordnet werden. Ferner ist es angenehm zu tragen und weist wenig Gewicht auf. Zudem ist es vorteilhaft, wenn das tragbare Element nicht stationär gebunden ist.

Erfindungsgemäß kann es vorgesehen sein, dass der Applikationsbereich binokular trennbar ist, wobei jedes Okular mindestens eine Lichtquelle, insbesondere LED, aufweist, die unabhängig voneinander Licht applizieren kann. Ebenfalls ist es denkbar, dass es sich um einen einzigen Applikationsbereich handelt, der mindestens eine Lichtquelle, insbesondere LED, aufweist. Zusätzlich oder alternativ ist es ebenfalls denkbar, dass das Steuergerät mindestens eine Lichtquelle, insbesondere LED, aufweist. Dabei kann das Licht von einem Steuergerät, insbesondere über einen optischen Leiter, zu dem Applikationsbereich, insbesondere zu den Okularen geleitet werden. Diese Weiterleitung des Lichtes kann dabei an jedes Okular oder in verschiedenen Bereichen des einen Applikationsbereiches unabhängig voneinander erfolgen. Insbesondere kann das System damit zur Applikation an jedem Auge eingesetzt werden. Über die optischen Strukturen können ferner zentrale neuronale Strukturen, wie bspw. des Gehirns und auch des Rückenmarks erreicht werden oder neuronale Strukturen, wie bspw. die Skelettmuskulatur. Vorteilhafterweise kann durch einen mindestens zweiteiligen Aufbau in Bezug auf die Okulare eine einfache Steuerung der Seitendifferenz hervorgerufen werden.

Es kann ebenfalls erfindungsgemäß vorgesehen sein, dass das Steuergerät in dem tragbaren Element integriert ist. Durch einen derartigen Einbau bzw. Integration ist das System einteilig ausgestaltet. Vorteilhafterweise ist es damit tragbar und flexibel einsetzbar. Insbesondere kann es vorgesehen sein, dass es sich bei dem tragbaren Element um eine Brille oder eine Manschette handelt. Das Steuergerät kann dementsprechend in der Brille oder Manschette verbaut sein. Insbesondere ist ein Einbau in das Halteelement, insbesondere einen der Bügel der Brille oder einen Gurt der Manschette von Vorteil. Ferner können ein Netzteil zur Stromversorgung und/oder Akkumulatoren vorgesehen sein. Vorteilhafterweise ist das System flexibler, wenn es sich um Akkumulatoren handelt, da es somit tragbar ausgestaltet sein kann und nicht stationär gebunden ist. Um einen Wechsel der Akkumulatoren bzw. ein Laden der Akkumulatoren zu umgehen ist ein Netzteil von Vorteil. Ferner kann eine externe Auswerteeinheit vorgesehen sein, die über eine feste Kabelverbindung und/oder eine Funkverbindung wie z.B. Bluetooth, NFC oder dergleichen verbunden sein kann. Durch eine möglichst einteilige Ausgestaltung des Systems sowie durch den Einsatz von bestimmten Materialen, wie bspw. Metall, Gummi und/oder Kunststoff oder dergleichen ist wenig Gewicht notwendig und das System ist besonders angenehm zu tragen.

Ferner ist es denkbar, dass das Steuergerät mit dem tragbaren Element in Signalverbindung steht, wobei insbesondere im Steuergerät eine Lichtquelle integriert ist. Die zumindest eine Lichtquelle kann dabei in dem Steuergerät eingebaut sein und lediglich über einen Lichtleiter das Licht zum tragbaren Element transportieren. Bei dem Lichtleiter kann es sich vorteilhafterweise um eine Signalverbindung insbesondere zur Übertragung der Lichtreize handeln. Die Signalverbindung kann diese über eine feste Kabelverbindung und/oder eine Funkverbindung, wie bspw. Bluetooth, NFC oder dergleichen übertragen. Falls die Lichtquelle im Steuergerät angeordnet ist kann dies weiterhin zu einer Reduzierung des Gewichts des tragbaren Elementes führen und damit ferner zu einem erhöhten Tragekomfort.

Ebenfalls wird die Aufgabe durch ein tragbares Element zur Stimulation, insbesondere zur Photostimulation, von neuronalen Strukturen zur physikalischen Beeinflussung der Aktivität der neuronalen Strukturen gelöst. Ferner umfasst das tragbare Element mindestens einen Applikationsbereich zur Applikation von Licht, vorzugsweise mindestens ein Steuergerät zur Steuerung und/oder Regelung von zumindest einer Lichtquelle zur Applikation von Licht sowie mindestens ein Halteelement zur Positionierung des tragbaren Elementes in räumlicher Nähe der neuronalen Strukturen. Bei der Stimulation kann es sich insbesondere um eine Photostimulation handeln. Eine Photostimulation kann durch Licht, insbesondere durch Lichtpulse und/oder Lichtblitze induziert werden. Die Photostimulation ist dabei als Provokationsmethode einsetzbar, bei welcher Lichtpulse und/oder Lichtblitze auf neuronale und/oder muskuläre Strukturen applizierbar sind. Bei dem Applikationsbereich kann es sich ferner um einen durchgehenden Bereich oder einen aus mehreren Teilbereichen, insbesondere Okularen, bestehenden Bereich handeln. Bei dem tragbaren Element kann es sich insbesondere um eine Brille handeln. Ebenfalls ist eine Manschette zur Anordnung an den neuronalen Strukturen denkbar. Der Applikationsbereich kann die neuronalen Strukturen zumindest teilweise abdecken. Bei den neuronalen Strukturen kann es sich insbesondere um optische Strukturen oder Zellen der Skelettmuskulatur handeln. Ebenfalls sind Dichtelemente denkbar, die den Applikationsbereich weiter abschirmen, so dass die neuronalen Strukturen keinen Umwelteinflüssen, insbesondere Licht, ausgesetzt sind. Der Applikationsbereich ist ferner insbesondere paarig ausgestaltet. Ferner weist das tragbare Element ein Halteelement auf, welches das tragbare Element am jeweiligen Applikationsort halten kann. Bei dem Halteelement kann es sich um einen oder mehrere Bügel handeln. Ebenfalls kann das Halteelement auch eine Struktur, wie ein Gummiband, einen Riemen oder ähnliches darstellen. Das Halteelement kann dafür sorgen, dass das tragbare Element sicher am Applikationsort gehalten wird. Vorzugsweise umfasst das tragbare Element ein Steuergerät. Dieses kann mit dem tragbaren Element verbunden sein und/oder mit diesem verbaut sein, so dass das Steuerelement in das tragbare Element integriert ist. Eine seitendifferente Applikation von Licht ist denkbar. Ferner ist auch eine sequenzielle Applikation möglich.

Ebenfalls wird die Aufgabe durch ein Verfahren zur Stimulierung, insbesondere zur Photostimulation, von neuronalen Strukturen zur physikalischen Beeinflussung der Aktivität der neuronalen Strukturen gelöst. Das System umfasst dabei mindestens ein Steuergerät zur Steuerung und/oder Regelung von zumindest einer Lichtquelle. Insbesondere kann ebenfalls ein tragbares Element zur Applikation von Licht vorgesehen sein. Das Verfahren weist dabei zumindest die Schritte (a) Erzeugen von Licht, (b) Regeln und/oder Steuern von Licht, (c) Applizieren von Licht, auf, wobei das Licht seitendifferent applizierbar ist.

Licht wird dabei insbesondere von einer Lichtquelle erzeugt. Die Lichtquelle kann dabei Licht in verschiedenen Wellenlängen, Frequenzen und Intensitäten erzeugen. Regelung bedeutet dabei Messen der zu beeinflussenden Größe (Regelgröße) in der Regelstrecke und kontinuierliches Vergleichen mit dem gewünschten Wert (Sollwert oder Führungsgröße). Der Regler kann entsprechend der Abweichung (Regeldifferenz) eine Stellgröße bestimmen, die so auf die Regelgröße einwirken kann, dass sie die Abweichung minimiert und die Regelgröße ein gewünschtes Zeitverhalten annimmt trotz vorhandener Störgrößen. Die Applikation von Licht erfolgt vorzugsweise zielgerichtet auf zumindest Teile der neuronalen Strukturen. Erfindungsgemäß ist das Licht dabei seitendifferent applizierbar. Bei einer seitendifferenten Applikation ist das Licht nicht gleichmäßig über eine gesamte Fläche applizierbar sondern vielmehr unterschiedlich an zumindest zwei Seiten. Die Seitendifferenz kann sich dabei in der Frequenz, der Intensität oder auch der Wellenlänge wiederspiegeln. Die Applikation kann dabei von nur einer oder auch von mehreren Lichtquellen herbeigeführt werden. Bei nur einer Lichtquelle ist es denkbar, dass verschiedene Hilfsmittel wie bspw. Spiegel oder Lichtleiter einsetzbar sind, damit die eine Lichtquelle an verschiedenen Seiten Licht unterschiedlich applizieren kann. Eine seitendifferente Applikation ist insbesondere bei paarigen neuronalen Strukturen denkbar, wie bspw. die Augen, die Nase, das Gehirn, das Rückenmark, die Rücken- und Halsmuskulatur oder die Extremitäten. Auch bei einheitlichen Strukturen ist eine seitendifferente Applikation denkbar, um diese an verschiedenen Stellen unterschiedlich zu behandeln.

Dabei kann es erfindungsgemäß vorgesehen sein, dass eine Messung der Aktivität von neuronalen Strukturen nach der Ausführung zumindest einem der Schritte (a) bis (c) durchführbar ist. Eine Messung kann dabei vorzugsweise über eine funktionelle Magnetresonanztomographie (fMRT) erfolgen. Dabei handelt es sich um ein bildgebendes Verfahren, um physiologische Funktionen im Inneren eines Organismus mit den Methoden der Magnetresonanztomographie darzustellen. Dabei können neuronale Strukturen mit hoher räumlicher Auflösung funktionell dargestellt werden. Vorzugsweise können die Messungen ebenfalls als elektroenzephalographische und/oder elektromyographische Messungen durchgeführt werden. Die Elektroenzephalografie (EEG) stellt eine Methode zur Messung von summierten elektrischen Aktivitäten neuronaler Strukturen, insbesondere des Gehirns, durch Aufzeichnungen von Spannungsschwankungen an der Kopfoberfläche dar, die insbesondere im Bereich von 5 bis 100 µV, aufgenommen werden. Vorteilhafterweise ist somit die Aktivität von neuronalen Strukturen, insbesondere die Aktivität des Gehirns in Echtzeit messbar und untersuchbar. Allgemein kann eine Lichtstimulation hinsichtlich ihres Effektes auf das Gehirn elektroenzephalographisch erfasst werden. Dabei kann vorteilhafterweise der Effekt auf das Gehirn, vorzugsweise auf frontale Regionen, elektroenzephalographisch (EEG) erfasst werden, der durch die Photostimulation hervorgerufen werden kann. Es können durch die Stimulation hervorgerufene Veränderungen in verschiedenen Frequenzspektren des EEG gemessen werden, insbesondere Veränderungen im Delta- (0-4 Hz), Theta- (4-8 Hz), Alpha- (8-12 Hz) und Beta-Frequenzbereich(12-20 Hz), vorzugsweise im Alpha-Frequenzbereich, hier vorzugsweise im Alpha1-Bereich (8-10 Hz) und im Alpha2-Bereich (10-12). Vorteilhafterweise können die EEG Messungen an allen Positionen des internationalen 10-20-Systems, vorzugsweise an den Positionen Fp1, Fp2, F3 und F4, erfolgen. Ferner ist es denkbar, dass die Amplituden und Frequenzen der elektroenzephalographisch (EEG) erfassbaren Wellen mit Hilfe einer Fourier Transformationsanalyse analysiert und sogenannte Power-Werte ermittelt werden können. Die EEG Messungen können dabei vor, während und nach einer Behandlungssitzung mit Photostimulation durchgeführt werden. Insbesondere können Vergleiche zwischen den Messwerten vor und nach einer Behandlungssitzung angestellt werden. Darüber hinaus ist es denkbar, dass die EEG Messungen auch im Zusammenhang mit anderen Formen der Neurostimulation als der Photostimulation durchgeführt werden können.

Ferner ist es denkbar, dass zumindest einer der Schritte (a) bis (c) an lebenden Organismen durchführbar ist. Damit ist es nicht nur möglich neuronale Strukturen in vitro sondern ebenfalls im lebenden Organismus, in vivo, messen zu können. Vorteilhafterweise sind somit Auswirkungen, und/oder Effekte, welche aus der Applikation von Licht resultieren direkt in Echtzeit sichtbar. Auch die verschiedenen Faktoren, wie Intensität, Wellenlänge oder Frequenz, können dadurch bei einer Applikation von Licht gegebenenfalls unmittelbar angepasst werden.

Ferner wird die Erfindung durch ein Computerprogramm für ein System zur Stimulierung, insbesondere zur Photostimulation, von neuronalen Strukturen zur physikalischen Beeinflussung der Aktivität der neuronalen Strukturen gelöst. Dabei ist es erfindungsgemäß vorgesehen, dass das Computerprogramm einen Algorithmus aufweist, der von einer Elektronikeinheit des Systems abgearbeitet wird. Der Algorithmus ist dabei vorteilhafterweise in das Verfahren gemäß einem der Ansprüche 11 bis 13 implementiert. Das Computerprogramm weist ferner eine entsprechende Software auf. Das Computerprogramm umfasst zumindest zwei bis beliebig viele, insbesondere 30 bis 100 Schritte. Zunächst wird zumindest eine Lichtquelle ausgewählt. Bei mehreren ausgewählten Lichtquellen können diese unterschiedliche Intensitäten aufweisen. Insbesondere umfasst das System drei Modi. Eine kontinuierliche Stimulation mit Lichtpulsen, eine Stimulation mit Lichtblitzen oder keine Stimulation. Dabei kann sowohl die Stimulationsdauer, als auch die Stimulationsintensität bzw. Lichtintensität, als auch die Frequenz der Lichtpulse bzw. Lichtblitze und die verschiedenen Seiten (links/rechts/oben/unten) variiert werden. Zusätzlich ist eine Dimm-Funktion möglich, wodurch eine Steigerung bzw. eine Abnahme der Lichtintensität von einem Ausgangswert bis zu einem Zielwert in einem vorgegebenen Zeitraum ermöglicht ist. Denkbar sind auch modulare Abfolgen von Stimulationsschritten, die sich in bestimmter Reihenfolge einander ablösen.

Ferner ist es denkbar, dass die Abfolge der Lichtstimulation in einem Regelkreis gesteuert und durch elektroenzephalographische (EEG) und/oder elektromyographische (EMG) Messwerte erfasst werden kann. Dabei ist es vorteilhaft, wenn die Abfolge der Lichtstimulation hinsichtlich der Parameter Lichtintensität, Dauer eines Lichtpulses mit konstanter Lichtintensität und Seite der Applikation des Lichtes an EEG- und EMG-Messwerten ausgerichtet werden kann. Bevorzugt handelt es sich dabei um durch Fourier Analyse ermittelte Werte. Sowohl die EEG-Werte als auch die EMG-Werte können untereinander in eine rechnerische Beziehung gesetzt werden. Hierzu werden vorteilhafterweise die EEG- und EMG-Messwerte in 32-, 64- oder 128-sekündigen Abständen über jeweils 4 oder 8 Sekunden ermittelt. Anschließend können Vergleiche aktueller zu unmittelbar vorangegangenen Messwerten angestellt werden. Dabei ist es denkbar, dass anzustrebende Zielgrößen bzw. Sollwerte der Messwerte vorgegeben werden können, die mit den tatsächlich gemessenen Werten bzw. Istwerten verglichen werden können. Die Abfolge der Lichtstimulation kann in der jeweiligen Epoche bis zur nächsten Erhebung der Messwerte derart geändert werden, dass die nächsten Messwerte näher an den Sollwerten liegen. Eine Epoche ist dabei vorteilhafterweise eine Stimulation vom Zeitpunkt des Endes der vorangegangenen Messung bis zum Beginn der aktuellen Messung. Das mit den Messwerten erfasste Ergebnis einer Epoche der Lichtstimulation kann mit dem Ergebnis der vorangegangenen Epoche verglichen werden. Die Lichtstimulation kann daraufhin vorteilhafterweise mit der Abfolge der Lichtstimulation der Epoche, deren Messwerte näher an den Zielwerten liegen, weitergeführt werden. Dieses Vorgehen kann bis zum Ende der Behandlungssitzung fortgeführt werden.

Weitere, die Erfindung verbessernde Maßnahmen ergeben sich aus der nachfolgenden Beschreibung zu einigen Ausführungsbeispielen der Erfindung, welche in den Figuren schematisch dargestellt sind. Sämtliche aus den Ansprüchen, der Beschreibung und den Zeichnungen hervorgehenden Merkmale und/oder Vorteile einschließlich konstruktiver Einzelheiten, räumliche Anordnungen und Verfahrensschritte, können sowohl für sich als auch in Kombination erfindungswesentlich sein. Dabei ist zu beachten, dass die Figuren nur beschreibenden Charakter haben und nicht dazu gedacht sind, die Erfindung in irgendeiner Form einzuschränken. Es zeigen:
- Fig. 1: schematische Darstellung eines Systems zur Stimulation von neuronalen Strukturen und
- Fig. 2: schematische Darstellung eines tragbaren Elementes zur Stimulation von neuronalen Strukturen

In den nachfolgenden Figuren werden für die gleichen technischen Merkmale, auch von unterschiedlichen Ausführungsbeispielen, die identischen Bezugszeichen verwendet.

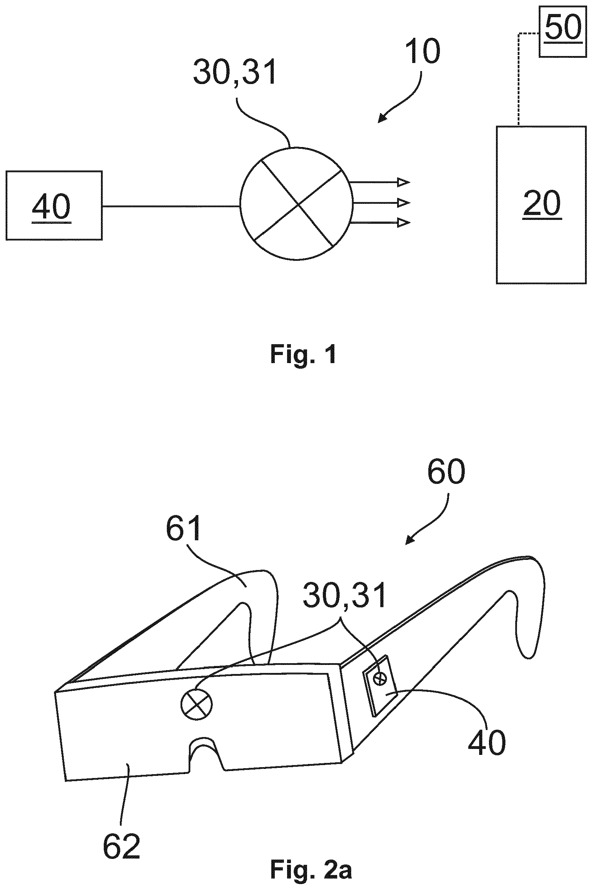

Figur 1 zeigt eine schematische Darstellung eines Systems 10 zur Stimulation von neuronalen Strukturen 20. Das System 10 weist dazu zumindest eine Lichtquelle 30 auf zur Applikation von Licht auf die neuronalen Strukturen. Ferner umfasst das System 10 ebenfalls ein Steuergerät 40 zur Steuerung und/oder Regelung von zumindest einer Lichtquelle 30. Bei der Lichtquelle 30 handelt es sich dabei vorzugsweise um zumindest eine LED 31. Bei der Stimulation handelt es sich damit vorteilhafterweise um eine Photostimulation mittels der zumindest einen Lichtquelle 30. Bei der Photostimulation handelt es sich vorzugsweise um Lichtpulse bzw. Lichtblitze von bevorzugt 0,1 bis 200 sec Dauer, besonders bevorzugt 5 bis 70 sec Dauer, mit einer Frequenz von insbesondere 0,05 bis 500 Hz, bevorzugt von 0,1 bis 200 Hz und besonders bevorzugt von 0,2 bis 100 Hz. Durch eine derartige Stimulation können die neuronalen Strukturen 20 aktiviert bzw. ihre Aktivität kann verändert werden. Dabei kann ebenfalls insbesondere ein Verfahren angewandt werden, bei welchem durch externe Stimulation eine Beeinflussung von neuronalen Strukturen hinsichtlich des Rhythmus ihrer elektrophysiologisch bzw. elektroenzephalographisch detektierbaren (Hirn-)Wellen (Summenpotentialschwankungen) und/oder elektromyographisch Signale erwirkt werden kann. Diese Stimulation kann bevorzugt über externe Reize, besonders bevorzugt durch Lichtpulse und/oder Lichtblitze, appliziert werden. Die Lichtpulse und/oder Lichtblitze können dabei bevorzugt eine dem Aktivitätsrhythmus der neuronalen Strukturen entsprechende Frequenz aufweisen, die beginnend mit einer niedrigen Frequenz von insbesondere 0,1 bis 50 Hz mit allmählich steigernder Frequenz bis zu insbesondere 50 bis 200 Hz beschleunigt und/oder beginnend mit einer hohen Frequenz von insbesondere 200 bis 5 Hz mit allmählich abnehmender Frequenz bis zu insbesondere 0,1 bis 20 Hz verlangsamt wird. Die neuronalen Strukturen 20 bestehen dabei vorzugsweise aus einem lebenden Organismus. Es kann sich aber ferner ebenfalls um isolierte neuronale Strukturen handeln.

Das Steuergerät 40 ist vorzugsweise ein elektronisches Modul, welches zumindest die Lichtquelle 30 steuert. Das Steuergerät 40 kann dabei insbesondere die Intensität, Frequenz und/oder die Wellenlänge der Lichtquelle 30 steuern. Die Wellenlänge umfasst dabei vorteilhafterweise Licht, insbesondere im Bereich von 300 nm bis 800 nm, bevorzugt im Bereich von 400 nm bis 700 nm, besonders bevorzugt im Bereich von 450 nm bis 600 nm. Bei einer Wellenlänge von 400 nm bis 700 nm wird im lebenden Organismus eines Säugetieres insbesondere der Nucleus suprachiasmaticus aktiviert. Die Beleuchtungsstärke kann eine Intensität von 100 bis 15000 Lux aufweisen, bevorzugt im Bereich von 500 Lux bis 12000 Lux, besonders bevorzugt im Bereich von 9000 Lux bis 10000 Lux. Auch ein Wechsel zwischen verschiedenen Intensitäten des Lichts ist möglich. Ferner sind wiederholte Stimulationen, bzw. sich wiederholende Stimulationsmuster, also verschiedene Abfolgen an Applikationen, denkbar. Ebenfalls ist es denkbar, dass die Lichtquelle 30 dimmbar sein kann. Dadurch können Vernetzungen der neuronalen Strukturen 20 verursacht werden. Ebenfalls ist es denkbar, dass sich durch eine derartige Stimulation bestimmte Vernetzungen neuronaler Strukturen 20 nicht ausbilden. Dadurch können verschiedene Indikationen der neuronalen Strukturen 20 behandelt werden. Vorteilhafterweise tritt durch den Gebrauch verschiedener Intensitäten keine Habituation der neuronalen Strukturen auf. Verschiedene Stimulationsmuster erreichen so verschiedene neuronale Strukturen und sind je nach zu behandelndem Krankheitsbild applizierbar bzw. anwendbar.

Erfindungsgemäß ist das Licht dabei seitendifferent applizierbar. Dabei wird das Licht nicht gleichmäßig über eine gesamte Fläche appliziert sondern vielmehr unterschiedlich an zumindest zwei verschiedenen Orten, insbesondere Seiten. Die Seitendifferenz kann sich dabei zumindest in der Frequenz, der Intensität oder auch der Wellenlänge unterscheiden. Eine seitendifferente Applikation kann dabei von nur einer oder auch von mehreren Lichtquellen 30 herbeigeführt werden. Bei nur einer Lichtquelle 30 ist es vorteilhaft, wenn Hilfsmittel wie bspw. Spiegel und/oder Lichtleiter eingesetzt werden, wodurch die Lichtquelle an verschiedenen Orten bzw. Seiten Licht unterschiedlich appliziert. Eine seitendifferente Applikation kommt dabei insbesondere bei paarigen neuronalen Strukturen in Frage. Dabei handelt es sich bspw. um Strukturen wie die Augen, die Nase, das Gehirn, das Rückenmark, die Rücken- und Halsmuskulatur oder Extremitäten. Auch bei einheitlichen Strukturen ist eine seitendifferente Applikation vorteilhaft, wenn keine gleichartige Applikation der neuronalen Struktur gewünscht ist. Bevorzugt wirkt eine Applikation von Licht auf oberflächennahe neuronale Strukturen, wie Haut, Nase oder Augen oder Skelettmuskulatur. Über nervöse Verschaltungen können jedoch auch zentrale neuronale Strukturen 20 wie das Gehirn oder das Rückenmark mittels einer Applikation von Licht erreicht werden. Insbesondere über das Auge ist es möglich bestimmte Regionen des Gehirns oder auch des Rückenmarks zu stimulieren. Durch eine seitendifferente Applikation ergeben sich ferner vielfältige verschiedene Möglichkeiten an Stimulationsmustern. Ferner ist es denkbar, dass mittels der Lichtquelle Licht applizierbar ist, wobei die Applikation kontinuierlich und/oder diskontinuierlich, insbesondere sequenziell, erfolgen kann. Durch verschiedenste Variationen der Applikation von Licht auf neuronale Strukturen, ist eine große Variation an Applikationsmöglichkeiten geschaffen. Vorteilhafterweise entsteht durch eine diskontinuierliche Applikation keine Habituation der neuronalen Strukturen, da verschiedene Stimulationsmuster erreicht werden können, die verschiedene neuronale Strukturen 20 aktivieren. Je nach zu behandelnder Indikation bzw. je nach zu behandelnden neuronalen Strukturen 20 kann ein anderes Muster der Applikation, bestehend aus zumindest Intensität, Frequenz oder Wellenlänge appliziert werden.

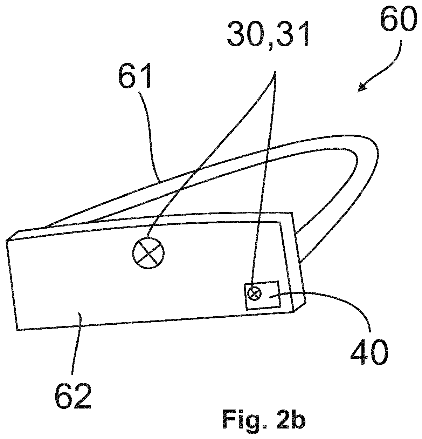

Figur 2a und 2b zeigen eine schematische Darstellung eines tragbaren Elementes 60. Dabei ist zumindest eine Lichtquelle 30 an und/oder im tragbaren Element 60 angeordnet. Das tragbare Element 60 umfasst dabei mindestens einen Applikationsbereich 62. Der Applikationsbereich 62 kann dabei aus einem durchgehenden Bereich oder aus mehreren Bereichen, insbesondere Okularen 62 bestehen. Ferner weist das tragbare Element 60 ebenfalls mindestens ein Halteelement 61 auf. Bei dem tragbaren Element 60 kann es sich insbesondere um eine Brille 60 handeln. Ebenfalls kann eine Manschette an neuronalen Strukturen 20 angeordnet werden. Durch den Applikationsbereich 62 sind neuronale, insbesondere optische Strukturen 20 oder Muskelzellen abdeckbar, so dass eine Applikation von Licht auf diese erfolgen kann. Ferner können Dichtelemente vorgesehen sein, die den Applikationsbereich weiter abschirmen, so dass die neuronalen Strukturen keinen Umwelteinflüssen, insbesondere Licht, ausgesetzt sind.

Da es sich bei den neuronalen Strukturen 20 vorzugsweise um paarige Strukturen handelt, ist der Applikationsbereich insbesondere paarig ausgestaltet. Wenn es sich bei dem tragbaren Element 60 um eine Brille 60 handelt, weist diese bevorzugt zwei Okulare 62 auf, wobei jedes Okular 62 vor einem Auge angeordnet sein kann. Ebenfalls kann es sich bei dem tragbaren Element 60 um eine Manschette 60 handeln. Ferner weist das tragbare Element 60 ein Halteelement 61 auf, welches das tragbare Element am jeweiligen Applikationsort halten kann. Bei dem Halteelement 61 kann es sich um einen oder mehrere Bügel, insbesondere bei einer Brille 60, handeln. Ebenfalls kann das Haltelement 61 auch eine Struktur, wie ein Gummiband, einen Riemen oder ähnliches darstellen. Das Halteelement 61 sorgt dafür, dass das tragbare Element 60 sicher am Applikationsort gehalten wird. Ferner kann es sich bei dem Steuergerät 40 um einzelne Elemente handeln, die mit dem tragbaren Element 60 verbunden sein können. Ein Verbauen mit dem tragbaren Element 60 ist ebenfalls denkbar. Es kann sich somit um ein einteiliges tragbares Element 60 oder auch um mehrere zusammensteckbare Teile handeln. Es ist ferner denkbar, dass das tragbare Element 60 an sich das Steuergerät 40 ist. Eine seitendifferente Applikation ist unabhängig vom tragbaren Element 60 bzw. vom Applikationsbereich, also unabhängig davon, ob dieser einteilig oder mehrteilig aufgebaut ist, möglich. Ferner ist auch eine sequenzielle Applikation möglich. Der Applikationsbereich kann dabei ebenfalls einen bestimmten Abstand zum Applikationsort aufweisen. Dieser Abstand kann insbesondere durch das Halteelement konstant gehalten werden. Ein bestimmter Abstand kann zusammen mit der Intensität, der Frequenz und der Wellenlänge wechseln.

Erfindungsgemäß kann es vorgesehen sein, dass der Applikationsbereich binokular trennbar ist. Jedes Okular 62 weist dabei mindestens eine LED 31 auf, die unabhängig voneinander Licht applizieren können. Insbesondere zur Applikation an jedem Auge kann das System 10 damit eingesetzt werden. Über die Augen können ferner zentrale neuronale Strukturen 20, wie bspw. des Gehirns und auch des Rückenmarks erreicht werden. Auch über andere periphere neuronale Strukturen 20 können zentrale neuronale Strukturen aktiviert bzw. stimuliert werden. Vorteilhafterweise kann durch einen mindestens zweiteiligen Aufbau in Bezug auf die Okulare 62 eine einfache Steuerung der Seitendifferenz hervorgerufen werden.

Das Steuergerät 40 kann in dem tragbaren Element 60 integriert sein, so dass das System 10 einteilig ausgestaltet ist. Es ist damit tragbar und flexibel einsetzbar. Das Steuergerät 40 kann dementsprechend in dem tragbaren Element 60 verbaut sein. Insbesondere ist ein Einbau des Steuergerätes 40 in das Halteelement 61 denkbar. Ein Einbau in einen der Bügel ist dabei von Vorteil, da eine derartige Position des Steuergerätes 40 den Tragekomfort nicht beeinflusst. Ferner können ein Netzteil und/oder Akkumulatoren zur Stromversorgung vorgesehen sein. Sowohl das tragbare Element 60 an sich also auch lediglich das Halteelement 61 können vorzugsweise aus bestimmten Materialen, wie bspw. Metall, Gummi und/oder Kunststoff oder dergleichen ausgestaltet sein um den Tragekomfort, insbesondere durch eine angenehme Haptik wie auch ein reduziertes Gewicht, zu verbessen.

Ferner ist es denkbar, dass das Steuergerät 40 mit dem tragbaren Element 60 in Signalverbindung steht. Die Signalverbindung kann ebenfalls insbesondere mit einer Lichtquelle 30 bestehen. Die zumindest eine Lichtquelle 30 kann dabei ebenfalls in dem Steuergerät 40 eingebaut sein. Ein optischer Leiter, insbesondere ein Lichtleiter, als Verbindung zwischen dem Steuergerät 40 und dem Applikationsbereich 62 ist ebenfalls denkbar. Bei dem optischen Leiter kann es sich vorteilhafterweise um eine Signalverbindung insbesondere zur Übertragung von Licht handeln. Die Signalverbindung kann dabei vorteilhafterweise über eine feste Kabelverbindung und/oder eine Funkverbindung wie z.B. Bluetooth, NFC oder dergleichen ausgebildet sein. Falls die Lichtquelle 30 im Steuergerät 40 angeordnet ist, kann dies weiterhin zu einer Reduzierung des Gewichts des tragbaren Elementes 60 führen und damit zu einem erhöhten Tragekomfort.

### Bezugszeichenliste

- 10: System
- 20: neuronale Struktur
- 30: Lichtquelle
- 31: LED
- 40: Steuergerät
- 50: Auswerteeinheit
- 60: tragbares Element
- 61: Halteelement
- 62: Applikationsbereich

## Patentansprüche

1. System (10) zur Stimulierung, insbesondere zur Photostimulation, von neuronalen Strukturen zur physikalischen Beeinflussung der Aktivität der neuronalen Strukturen (20), umfassend mindestens eine Lichtquelle (30) und ein Steuergerät (40) zur Steuerung und/oder Regelung von zumindest der Lichtquelle (30) zur seitendifferenzierten Applikation von Licht,
**dadurch gekennzeichnet, dass** es sich bei der Photostimulation um Lichtpulse handelt, von 5 bis 200 sec Dauer, wobei mehrere neuronale Strukturen gleichzeitig stimulierbar sind.

2. System (10) gemäß Anspruch 1,
**dadurch gekennzeichnet, dass**
die zumindest eine Lichtquelle (30) mindestens eine LED (31) umfasst.

3. System (10) gemäß einem der vorhergehenden Ansprüche,
**dadurch gekennzeichnet, dass**
mittels der Lichtquelle (30) Licht applizierbar ist, wobei die Applikation kontinuierlich und/oder diskontinuierlich, insbesondere sequenziell, erfolgen kann.

4. System (10) gemäß einem der vorhergehenden Ansprüche,
**dadurch gekennzeichnet, dass**
das Licht eine Wellenlänge im Bereich von 300 nm bis 800 nm, bevorzugt im Bereich von 400 nm bis 700 nm, besonders bevorzugt im Bereich von 450 nm bis 600 nm, umfasst.

5. System (10) gemäß einem der vorhergehenden Ansprüche,
**dadurch gekennzeichnet, dass**
die Lichtquelle (30) eine Beleuchtungsstärke von 100 bis 15000 Lux aufweist, bevorzugt im Bereich von 500 Lux bis 12000 Lux, besonders bevorzugt im Bereich von 9000 Lux bis 10000 Lux, umfasst.

6. System (10) gemäß einem der vorhergehenden Ansprüche,
**dadurch gekennzeichnet, dass**
die neuronalen Strukturen (20) neuronales Gewebe aufweisen, insbesondere humane neuronale Zellen.

7. System (10) gemäß einem der vorhergehenden Ansprüche,
**dadurch gekennzeichnet, dass**
die neuronalen Strukturen (20) Muskelzellen aufweisen, insbesondere humane Zellen der Skelettmuskulatur.

8. System (10) gemäß einem der vorhergehenden Ansprüche,
**dadurch gekennzeichnet, dass**
zumindest eine Lichtquelle (30) an einem tragbaren Element (60) angeordnet ist, wobei das tragbare Element (60) mindestens einen Applikationsbereich (62), insbesondere mit zwei Okularen (62), und mindestens ein Halteelement (61) aufweist.

9. System (10) gemäß Anspruch 8,
**dadurch gekennzeichnet, dass**
der Applikationsbereich (62) binokular trennbar ist, wobei jedes Okular (62) mindestens eine Lichtquelle (30), insbesondere eine LED (31), aufweist, wobei Licht jeder Lichtquelle (30) unabhängig voneinander applizierbar ist und/oder wobei das Steuergerät (40) mindestens eine Lichtquelle (30), insbesondere LED (31), aufweist, wobei vom Steuergerät (40) Licht auf jedes Okular (62) unabhängig voneinander applizierbar ist.

10. System (10) gemäß einem der vorhergehenden Ansprüche 8 oder 9,
**dadurch gekennzeichnet, dass**
das Steuergerät (40) in dem tragbaren Element (60) integriert ist.

11. System (10) gemäß einem der vorhergehenden Ansprüche,
**dadurch gekennzeichnet, dass**
das Steuergerät (40) mit dem tragbaren Element (60) in Signalverbindung steht, wobei insbesondere im Steuergerät (40) zumindest eine Lichtquelle (30) integriert ist.

12. Verfahren zur nicht therapeutischen Stimulierung, insbesondere zur Photostimulation, von neuronalen Strukturen (20) zur physikalischen Beeinflussung der Aktivität der neuronalen Strukturen (20) aufweisend zumindest die folgenden Schritte:
a) Erzeugen von Licht
b) Regeln und/oder Steuern von Licht
c) Applizieren von Licht,
wobei das Licht seitendifferent appliziert wird, wobei es sich bei der Photostimulation um Lichtpulse handelt, von 5 bis 200 sec Dauer, wobei mehrere neuronale Strukturen gleichzeitig stimuliert werden können.

13. Verfahren gemäß Anspruch 12,
**dadurch gekennzeichnet, dass**
eine Messung der Aktivität von neuronalen Strukturen nach der Ausführung zumindest einer der Schritte (a) bis (c) durchgeführt wird.

14. Verfahren gemäß Anspruch 12 oder 13,
**dadurch gekennzeichnet, dass**
zumindest einer der Schritte a) bis c) an lebenden Organismen durchgeführt wird.

15. Computerprogramm für ein System (10) zur Stimulierung, insbesondere zur Photostimulation, von neuronalen Strukturen zur physikalischen Beeinflussung der Aktivität der neuronalen Strukturen,
**dadurch gekennzeichnet, dass**
das Computerprogramm einen Algorithmus aufweist, der von einer Elektronikeinheit des Systems (10) abgearbeitet wird, wobei der Algorithmus das Verfahren gemäß einem der Ansprüche 12 bis 14 implementiert.

## Claims

1. System (10) for stimulating, in particular photostimulating, neuronal structures for physically influencing the activity of the neuronal structures (20), comprising at least one light source (30) and a control unit (40) for controlling and/or regulating at least the light source (30) for the application of light,
**characterized in that**
photostimulation comprises a light pulse of essentially 5 to 200 seconds duration, wherein several neuronal structures can be stimulated simultaneously.

2. System (10) according to claim 1,
**characterized in that**
the at least one fight source (30) comprises at least one LED (31).

3. System (10) according to one of the preceding claims,
**characterized in that**
light can be applied by means of the light source (30), wherein the application can be continuous and/or discontinuous, in particular sequential.

4. System (10) according to one of the preceding claims,
**characterized in that**
the light comprises a wavelength essentially in the range from 300 nm to 800 nm, preferably in the range from 400 nm to 700 nm, particularly preferably in the range from 450 nm to 600 nm.

5. System (10) according to one of the preceding claims,
**characterized in that**
the light source (30) comprises an illuminance of essentially 100 to 15000 lux, preferably in the range from 500 lux to 12000 lux, particularly preferably in the range from 9000 lux to 10000 lux.

6. System (10) according to one of the preceding claims,
**characterized in that**
the neural structures (20) comprise neural tissue, in particular human neural cells.

7. System (10) according to one of the preceding claims,
**characterized in that**
the neuronal structures (20) comprise muscle cells, in particular human skeletal muscle cells.

8. System (10) according to one of the preceding claims,
**characterized in that**
at least one light source (30) is arranged on a portable element (60), the portable element (60) comprising at least one application area (62), in particular with two eyepieces (62), and at least one holding element (61).

9. System (10) according to claim 8,
**characterized in that**
the application area (62) can be separated binocularly, wherein each eyepiece (62) has at least one light source (30), in particular an LED (31), wherein light from each light source (30) can be applied independently of one another and/or wherein the control unit (40) comprises at least one light source (30), in particular an LED (31), wherein light can be applied to each eyepiece (62) independently of one another by the control unit (40).

10. System (10) according to one of the preceding claims 8 or 9,
**characterized in that**
the control unit (40) is integrated in the portable element (60).

11. System (10) according to one of the preceding claims,
**characterized in that**
the control unit (40) is in signal connection with the portable element (60), wherein in particular at least one light source (30) is integrated in the control unit (40).

12. Method for the non-therapeutic stimulation, in particular photostimulation, of neuronal structures (20) for physically influencing the activity of the neuronal structures (20), comprising at least the following steps:
a) Generating light
b) Regulation and/or control of light
c) Application of light,
wherein the light is applied in a side-differentiated manner, wherein photostimulation being light pulses of essentially 5 to 200 sec duration, wherein several neuronal structures can be stimulated simultaneously.

13. Method according to claim 12,
**characterized in that**
a measurement of the activity of neuronal structures is carried out after the execution of at least one of the steps (a) to (c).

14. Method according to claim 12 or 13,
**characterized in that**
at least one of steps (a) to (c) is carried out on living organisms.

15. Computer program for a system (10) for the stimulation, in particular photostimulation, of neuronal structures for physically influencing the activity of the neuronal structures
**characterized in that**
the computer program comprises an algorithm which is processed by an electronic unit of the system (10), the algorithm implementing the method according to one of claims 12 to 14.

## Revendications

1. Système (10) de stimulation, en particulier de photostimulation, de structures neuronales pour influencer physiquement l'activité des structures neuronales (20), comprenant au moins une source lumineuse (30) et un dispositif de commande (40) pour commander et/ou réguler au moins la source lumineuse (30) pour l'application de la lumière,
**caractérisé en ce que**
la photostimulation est une impulsion lumineuse d'une durée essentiellement de 5 à 200 secondes, dans laquelle plusieurs structures neuronales peuvent être stimulées simultanément.

2. Système (10) selon la revendication 1,
**caractérisé en ce que**
la source lumineuse (30) comprend au moins une LED (31).

3. Système (10) selon l'une des revendications précédentes,
**caractérisé en ce que**
la lumière peut être appliquée au moyen de la source lumineuse (30), l'application pouvant être continue et/ou discontinue, en particulier séquentielle.

4. Système (10) selon l'une des revendications précédentes,
**caractérisé en ce que**
la lumière comprend une longueur d'onde comprise entre 300 nm et 800 nm, de préférence entre 400 nm et 700 nm, et plus particulièrement entre 450 nm et 600 nm.

5. Système (10) selon l'une des revendications précédentes,
**caractérisé en ce que**
la source lumineuse (30) comprend une luminosité essentiellement de 100 à 15000 lux, de préférence dans la plage de 500 lux à 12000 lux, particulièrement préférablement dans la plage de 9000 lux à 10000 lux.

6. Système (10) selon l'une des revendications précédentes,
**caractérisé en ce que**
les structures neuronales (20) comprennent le tissu neural, en particulier les cellules neuronales humaines.

7. Système (10) selon l'une des revendications précédentes,
**caractérisé en ce que**
les structures neuronales (20) comprennent les cellules musculaires, en particulier les cellules musculaires squelettiques humaines.

8. Système (10) selon l'une des revendications précédentes,
**caractérisé en ce que**
au moins une source lumineuse (30) est disposée sur un élément portable (60), l'élément portable (60) présentant au moins une zone d'application (62), en particulier avec deux oculaires (62), et au moins un élément de maintien (61).

9. Système (10) selon la revendication 8,
**caractérisé en ce que**
la zone d'application (62) peut être séparée de manière binoculaire, dans lequel chaque oculaire (62) présente au moins une source lumineuse (30), en particulier une LED (31), dans lequel la lumière de chaque source lumineuse (30) peut être appliquée indépendamment l'une de l'autre et/ou dans lequel le dispositif de commande (40) présente au moins une source lumineuse (30), en particulier une LED (31), dans lequel la lumière peut être appliquée sur chaque oculaire (62) indépendamment l'une de l'autre par le dispositif de commande (40).

10. Système (10) selon l'une des revendications précédentes 8 ou 9,
**caractérisé en ce que**
le dispositif de commande (40) est intégrée dans l'élément portable (60).

11. Système (10) selon l'une des revendications précédentes,
**caractérisé en ce que**
le dispositif de commande (40) est en liaison de signal avec l'élément portable (60), dans lequel en particulier au moins une source lumineuse (30) est intégrée dans le dispositif de commande (40).

12. Procédé de stimulation non thérapeutique, en particulier de photostimulation, des structures neuronales (20) pour influencer physiquement l'activité des structures neuronales (20), comprenant au moins les étapes suivantes :
a) Produire de la lumière
b) Régulation et/ou contrôle de la lumière
c) Application de la lumière,
dans lequel la lumière est appliquée de manière différenciée, la photostimulation comprenant des impulsions lumineuses d'une durée essentiellement de 5 à 200 secondes, ce qui permet de stimuler simultanément plusieurs structures neuronales.

13. Procédé selon la revendication 12,
**caractérisé en ce que**
une mesure de l'activité des structures neuronales est effectuée après l'exécution d'au moins une des étapes (a) à (c).

14. Procédé selon la revendication 12 ou 13,
**caractérisé en ce que**
au moins une des étapes (a) à (c) est effectuée sur des organismes vivants.

15. Programme informatique pour un système (10) de stimulation, en particulier de photostimulation, des structures neuronales pour influencer physiquement l'activité des structures neuronales
**caractérisé en ce que**
le programme informatique comprend un algorithme qui est traité par une unité électronique du système (10), l'algorithme mettant en œuvre la méthode selon l'une des revendications 12 à 14.
